# EUROPEAN PATENT APPLICATION

(11) **EP 4 571 244 A1**
(43) Date of publication of application: **18.06.2025**
(21) Application number: 24213164.7
(22) Date of filing: 15.11.2024
(51) Int. Cl.: G01B 11/16, A61B 34/30

(54) **ROBOT SENSING**

(30) Priority: 15.12.2023 GB 202319251
(71) Applicant: Rolls-Royce plc, London N1 9FX (GB)
(72) Inventor: Wild, Samuel P, Derby, DE24 8BJ (GB); Dong, Xin, Derby, DE24 8BJ (GB); Norton, Andrew D, Derby, DE24 8BJ (GB)
(74) Representative: Rolls-Royce plc

(57) **Abstract**

A fibre system for determining the shape of a flexible object, the system comprising having at least one coated optical fibre, the coated optical fibre having at least one optical fibre having at least one portion comprising a periodic distributed Bragg reflectorwithin the fibre, the at least one optical fibre being placed in and with a first flexible tube positioned around, and wherein the first flexible tube further has a first coating positioned around it.

## Description

### Overview of the disclosure

The disclosure relates to the integration of fibre Bragg gratings into flexible arm robots, such as borescopes and/or continuum arm, or snake robots and for any associated passive section. The disclosure further relates to a fibre Bragg grating structure for use in a flexible arm robot.

### Background of the disclosure

Flexible robotic arm systems such as continuum arm robots, borescopes, snake arm robots and endoscopes are used in a number of areas of industry from repair of complex equipment to performing medical operations. The reason that they are used is because their controllable flexibility allows them to safely access spaces that would be inaccessible without much more extensive work. Flexible robotic arms can be used for both inspection and repair processes, with the nature of the probe being determined by the tool or camera system that is used as the end effector on the robot. With these benefits the uses for flexible robotic arms are going to have a greater number of applications.

Despite the number of applications there are still issues with the use of flexible arm robots. Many of the issues are also related to their advantage in the that their flexibility means that positional control is not very accurate; this means that the robot cannot easily be controlled automatically. As such, the systems require complex systems in place to determine the position and shape of the robot such that a task can be performed. Due to the difficulty in knowing the exact shape of the robot within the workplace it is difficult to determine an accurate length of robotic arm that needs to be inserted into the workspace. Therefore, it is important to develop systems that can allow the shape of the robotic arm to be determined.

### Summary of the Disclosure

The scope of the disclosure is set out in the appended claims.

According to a first aspect of the disclosure there is presented a fibre system for determining the shape of a flexible object, the system comprising having at least one coated optical fibre, the coated optical fibre having at least one optical fibre having at least one portion comprising a periodic Bragg grating within the fibre, the at least one optical fibre being placed in and with a first flexible tube positioned around, and wherein the first flexible tube further has a first coating positioned around it.

A second flexible tube may be positioned around the first coating and around the second flexible tube has a second coating is positioned around.

There may be more than one coated optical fibre within the fibre system.

The first flexible tube may be made from a shape memory alloy or a superelastic material.

The first coating may be made from at least one of: PTFE, graphene, thin carbon material, low-friction gel, low-friction fluid or a bearing system..

The second or subsequent flexible tubes may be made from a shape memory alloy or a superelastic material.

The second coating may be made from at least one of: PTFE, graphene, thin carbon material, low-friction gel, low-friction fluid or a bearing system.

The first and/or second coating may be directly applied to the first and/or second flexible tube.

The first and/or second coating may be a thin film applied over the first and/or second flexible tube.

Surrounding the at least one coated optical fibre may be a flexible support structure.

The flexible support structure may be made from silicone or a flexible resin.

The tolerance between the fibre Bragg optical fibre and the first flexible tube may be between 0.005 and 0.5 mm.

According to a second aspect of the disclosure there is presented a flexible arm robot system comprising an arm having at least a first and second section linked by a joint, the joint being coupled to actuators to adjust the angle of the first section relative to the second section, and wherein through the first section and second section a fibre system according to the first aspect is passed, and wherein the fibre system is coupled to an interrogator.

There may be provided a plurality of sections of fibre system and between each section of fibre system may be provided a bush.

The bush may be made from at least one of: Nitinol, copper, aluminium, plastic materials, 3D printable materials such as liquid resin materials or thermoplastics.

### The fibre may be integrated into the inner or outer structure of the flexible arm

The skilled person will appreciate that except where mutually exclusive, a feature described in relation to any one of the above aspects may be applied mutatis mutandis to any other aspect. Furthermore, except where mutually exclusive any feature described herein may be applied to any aspect and/or combined with any other feature described herein.

### Brief Description of the Drawings

Embodiments will now be described by way of example only with reference to the accompanying drawings, which are purely schematic and not to scale, and in which:
FIG. 1A presents a prior art example of a cut away of a continuum arm robot;
FIG. 1B shows an example of the joints of a continuum arm robot;
FIG. 2 presents an example of an optical fibre with a Bragg reflector constructed within a short segment of it;
FIG. 3 presents an example of a mounting for a fibre Bragg grating according to the present disclosure;
FIG. 4 presents an alternative example of an FBG according to the present disclosure;
FIG. 5 presents a side view of the of the use of a Fibre Bragg gratings in a continuum arm robot;
FIG. 6 shows a schematic of the integration of the fibre Bragg grating into a continuum arm robot.

### Detailed Disclosure

Aspects and embodiments of the present disclosure will now be discussed with reference to the accompanying figures. Further aspects and embodiments will be apparent to those skilled in the art.

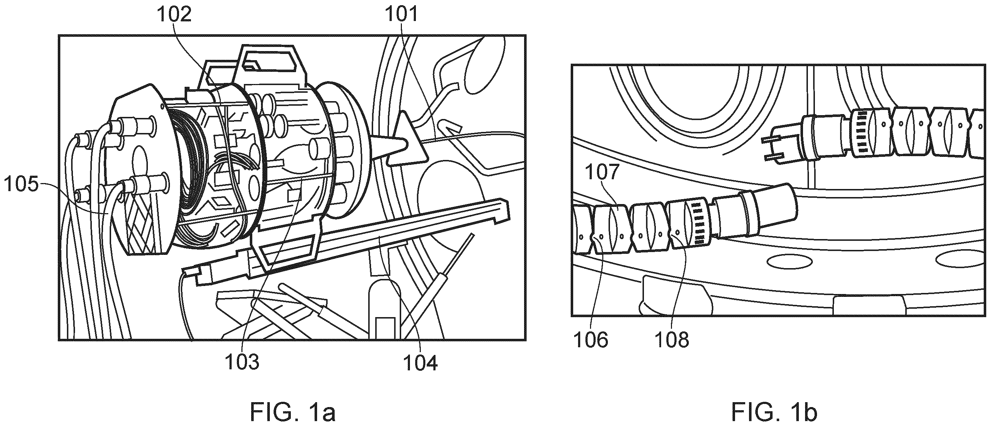

FIG. 1A presents a prior art example of a cut away of a continuum arm robot. The prior art continuum arm robot comprises the continuum arm robot portion 101 permanently integrated and extending out from the actuator pack 102. The actuator pack 102 contains a plurality of independent actuators 103. These actuators are used to modulate the tension within the tendons that run through the continuum arm 101. The tendons are associated with joints within the arm; each of these joints is designed to move in response to a tensioning or relaxing of the tendon associated with the joint. This tensioning or relaxing of the tendon therefore causes a contraction or extension of the joint, which allows the continuum arm to bend. The actuator pack is shown being positioned on a rail or support 104, which is positioned close to the component to be inspected. The actuator is also provided with a plurality of power and signal cables 105 that are used to power and address the actuators. The individual signals across the range of actuators provide control of the joints such that the continuum arm 101 can be directed. Not shown in FIG. 1A is that there is also a need for an operator with a computing device that is linked to the actuator to control movement of the continuum arm and to perform the desired task. As the continuum arm is permanently integrated into the actuator pack if a different tool is required it requires the use of a complete continuum arm robot system including the actuator. The computing device that is connected to the prior art actuator may be any suitable computing system such as a laptop computer featuring the requisite operating software for the robot and a control input such as a joystick, which allows the continuum arm to be controlled.

FIG. 1B shows an example of the joints of a continuum arm robot. The arm comprises multiple joints, which require at least 2 cables per joint. For example, a system having three joints, each having 4 tendons per joint will require 12 actuators to drive. To increase the number of joints either the number of actuators needs to be increased or the number of tendons per joint needs to be reduced. Highlighted joints 106, 107, 108 are able to be manipulated to move in three dimensions. The joints are configured so that joints 106 and 108 are able to be able to flex in the same plane relative to the centre of the arm, whilst the plane of that joint 107 is able to move in is offset by 90° to joints 106 and 108. It is through this repeating configuration of alternating joint angles, each of which results in the movement in different orthogonal plane, that allows the arm to be manipulated in three dimensions. Each joint within the arm has a limit to the amount they are able to flex; this is defined by the design of the arm and the materials that are used. The limit of flex in each joint sets characteristics such as the minimum bending radius and the requirements for the torque that is required to cause a resultant change within the joint. It is the presence of the space in the joints that allows the joint to move and the ease of movement of the joint that results in a low stiffness of the arms in comparison to other robotic arms of the same length. This is because the structural behaviour of a snake-like robotic manipulator can be compared to a cantilever beam under load; this is because the system is fixed on one end to the base with actuation pack and the remainder of the arm is used to navigate through the environment without other points of contact. In this condition, every load applied on the body and/or the tip of the snake-like robot, including its own weight, imposes a significant deflection from the ideal position. At the end of the arm there is positioned a tool or probe that is designed to perform one or more functions once the continuum arm is in position. The heads of the continuum arm robots are often provided with optical systems so that the operator is able to view the head as it is being inserted into the component and to control the head as it performs its tasks. The optical system is also frequently coupled to an illumination system. The control cables for the tool, electrical power connectors to the illuminations system and optical cables usually are able to run through the centre of the joints within the continuum arm. This has the benefit of protecting the cables form any potential damage. Although the above description is for a continuum arm robot, it will be appreciated that the disclosure is also applicable to other flexible robotic arm systems, such as borescopes and endoscopes.

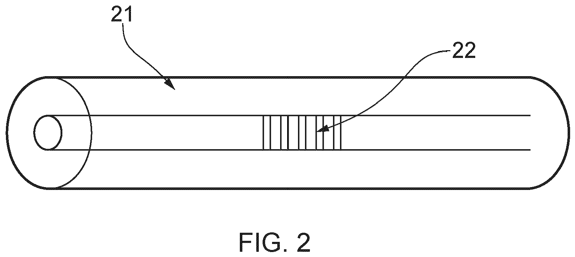

Fibre Bragg gratings have been used in flexible robotic systems to be able to determine the amount of deflection within the system. FIG. 2 presents an example of an optical fibre 21 with a Bragg reflector 22 constructed within a short segment of it; this is also known as a Fibre Bragg grating structure (FBG). The Bragg reflector allows certain wavelengths to transmitted and others to be reflected. FBG shape sensors are optical fibres that can measure an object's shape based on the strain and curvature of each grating. FBG shape sensrs can be multicore or multiple single core fibres. As light passes through the optical fibre, it is reflected at each grating at a specific wavelength called the Bragg wavelength. The shift in the Bragg wavelengths can be used to calculate the curvature, and a series of curvatures can then be used to determine the overall shape. Although there have been limited application of Bragg fibres in sensing the shape of hyper-redundant robots there are still issues in their use as they are unable to detect any twist within the fibre, as such the exact position cannot be known precisely. The lack of accuracy in positioning reduces the usability of continuum robots as they cannot be used in any situation in which they can potentially damage the workspace.

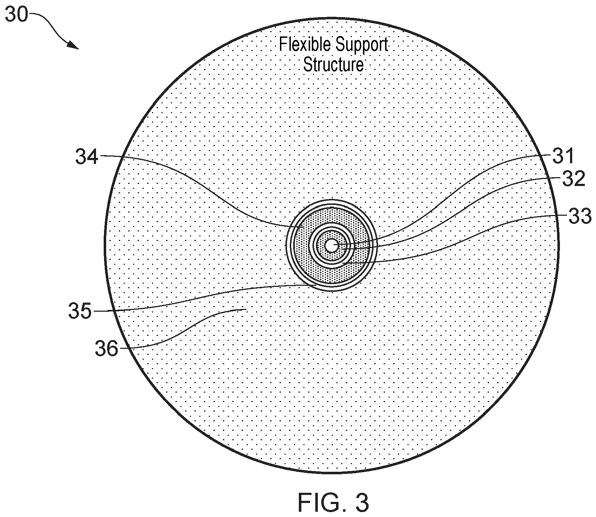

FIG. 3 presents an example of a mounting for a fibre Bragg grating 30 according to the present disclosure. In this the fibre Bragg grating 31 is placed in a first flexible tube 32. The first flexible tube may be made from a shape memory alloy (e.g., Nitinol (NiTiO) Fe -Mn-Si, Cu-Zn-Al and Cu-Al-Ni) or any supereleastic material (e.g., nitinol). The tube has a small tolerance between the fibre and the tube. The tolerances may be between 0.005 and 0.5 mm. Around the first flexible tube is positioned a first coating film 33 with a low frictional coefficient. The first coating film may be a coating on the first flexible tube. Alternatively, it can be a separate thin film. The first thin film may be made from PTFE, graphene, thin carbon material, low-friction gel, low-friction fluid or a bearing system.. Alternatively, it the first coating may be a lubricant such as a silicone coating. A second flexible tube 34 then positioned around the outside of the first thin film coating. The second flexible tube can be made from a shape memory alloy (e.g., Nitinol (NiTiO) Fe -Mn-Si, Cu-Zn-Al and Cu-Al-Ni) or any supereleastic material (e.g., nitinol). Around the second flexible tube is positioned a second flexible film 35. This film can be a coated layer or a separate thin film. The second coating may be made from the same material as the first coating or may be made from a different material. The second coating may be made from PTFE, graphene, thin carbon material, low-friction gel, low-friction fluid or a bearing system. Surrounding this core structure is a flexible support structure 36. The support structure may made from one of the following: silicone, flexible resin, latex, rubber, PVC, TPE or polyurethane. The flexible support structure may be moulded or created using three-dimensional printing techniques.

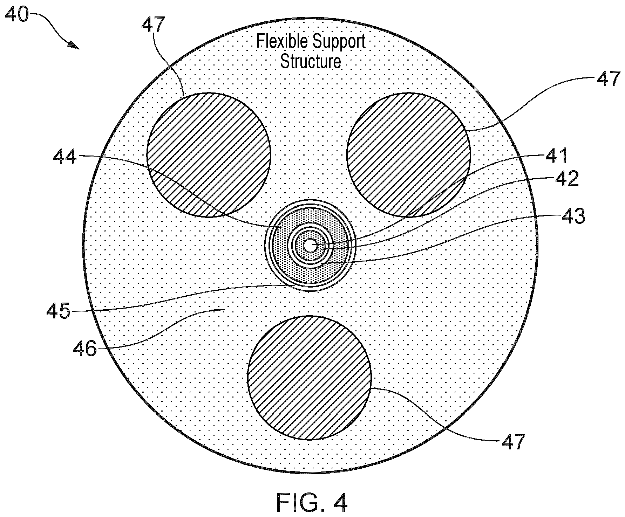

FIG. 4 presents an alternative example of an FBG system 40 to that presented in FIG. 3. In this, the fibre Bragg grating 41 is placed in a first flexible tube 42. The first flexible tube may be made from a shape memory alloy (e.g., Nitinol (NiTiO) Fe -Mn-Si, Cu-Zn-Al and Cu-Al-Ni) or any supereleastic material (e.g., nitinol)The tube has a small tolerance between the fibre and the tube. The tolerances may be between 0.005 and o.5 mm. Around the first flexible tube is positioned a first thin film 43 with a low frictional coefficient. The first coating may be a coating on the first flexible tube or a separate thin film. The first coating may be made from PTFE, graphene, thin carbon material, low-friction gel, low-friction fluid or a bearing system. A second flexible tube 44 is then positioned around the outside of the first coating. The second flexible tube can be made from a shape memory alloy (e.g., Nitinol (NiTiO) Fe -Mn-Si, Cu-Zn-Al and Cu-Al-Ni) or any supereleastic material (e.g., nitinol). Around the second flexible tube is positioned a second coating 45. This coating can be a coated layer or a thin film. The second coating may be made from the same material as the first coating or may be made from a different material. The second thin film may be made from PTFE, graphene, thin carbon material, low-friction gel, low-friction fluid or a bearing system. Surrounding this core structure is a flexible support structure 46. The support structure may made form silicone, or a flexible resin. The difference between FIG. 3 and FIG. 4 is that in FIG. 4 there is more than one cavity 47 within the flexible support structure. FIG. 4 presents the use of three cavities. There may be any suitable number of cavities with the support structure. For example, there may be between 1-6 cavities present with the flexible support structure. The flexible support structure may be moulded or created using three-dimensional printing techniques.

Although the examples shown in FIG. 3 and FIG. 4 there are two flexible tubes surrounding the fibre. However, there only needs to be a single flexible tube with a first thin film present with the flexible support structure. The presence of the at least one flexible tube with the at least one thin film coating it, means that the support structure allows the core to act akin to a roller bearing and thus preventing, or at least minimising, the effect of twist on the fibre. Without the presence of twist, the angular shape can be better determined, and the shape of the continuum robot can be determined with improved accuracy. Consequently, this increases the positional accuracy of the continuum arm robot.

The larger outer tube with transfer film, or other low friction solution, wrapped either side, allows the larger outer tube to act as a roller bearing interface between inner tube and the flexible support structure. The flexible support structure inside of the continuum robot takes most of the cross-sectional space can be manufactured from a material with minimal plastic deformation. As such, it would not create much additional stiffness down the central channel.

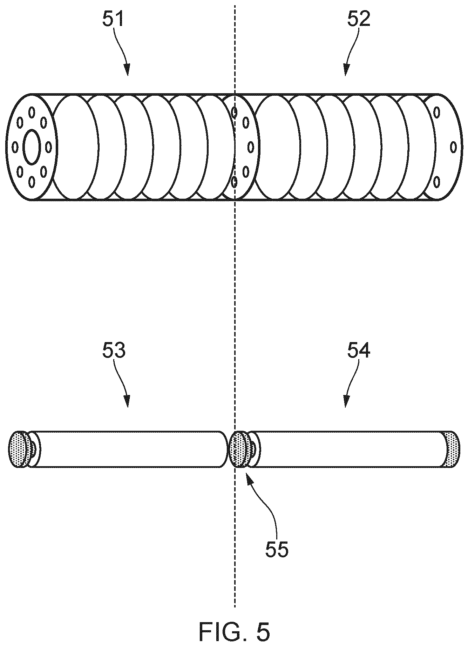

FIG. 5 presents a side view of the of the use of a Fibre Bragg gratings in a continuum arm robot. FIG. 5 shows a pair of sections of continuum arm 51 and 52. The segments represent a fixed number of sections having joints between them. For example, this could be one, two, three, four or five sections. Each section, as shown in FIG. 5, may be provided with a length of optical fibre 53 and 54 containing a Bragg fibre grating as discussed in relation to FIG. 3 and FIG. 4. Alternatively, the optical fibre containing the Bragg fibre grating may be positioned in alternate sections of the continuum arm. As shown in FIG. 5 between each section of optical fibre containing a Bragg optical grating is a bush 55. The bush may have the same diameter as the optical fibre. The bush may be made from Nitinol or a material such as aluminium or copper or plastic material, perhaps 3D printable, such as liquid resin materials or thermoplastics. The bush is used to isolate the different sections of the optical fibre gratings having the Bragg gratings, so that each sections curvature can be determined without any effects of twist propagating along the length of the continuum arm, which can be detrimental to determination of curvature. Also, continuum robots have the characteristic that every active section displays different twisting, so separating each section with a separate FBG benefits from being able to detect its own curvature without the twist. It is possible that only a single optical fibre with a fibre Bragg grating incorporated, the fibre may be positioned at any suitable position along the continuum robot arm.

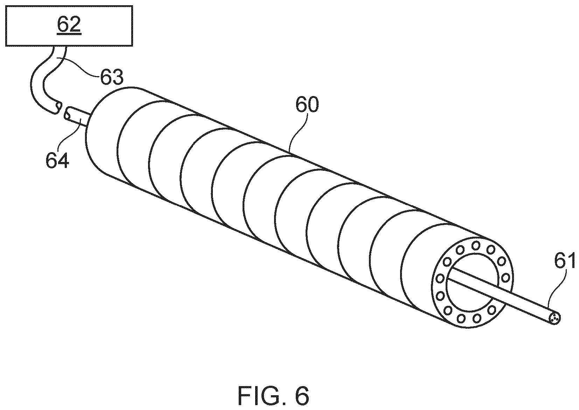

FIG. 6 shows a schematic of the integration of the fibre Bragg grating 61 into a continuum arm robot 60. The fibre (single or multicore) or fibres that house the Bragg gratings are connected to an interrogator 62. The interrogator is used to monitor the change in the wavelength of light that is transmitted along the optical fibre. From the change in wavelength that is detected by the interrogator the shape of the system can be determined. The fibre or fibres have a lead length 63 that is associated with the length of passive section of the continuum robot, that is to say the length of the continuum arm robot whose shape is not mechanically deformable. The first Bragg grating 64 is positioned at the base of the continuum robot. Every further joint may have a fibre having a Bragg grating corresponding to the position of the joint. Alternatively, the Bragg gratings may be positioned at alternative joints in the system. In this image the fibre Bragg system is shown running down the centre of the continuum robot. However, the fibres could be positioned in the outer walls of the continuum robot or borescope tool, including attached to the other circumference of the tool.

It will be understood that the invention is not limited to the embodiments above-described and various modifications and improvements can be made without departing from the concepts described herein. Except where mutually exclusive, any of the features may be employed separately or in combination with any other features and the disclosure extends to and includes all combinations and sub-combinations of one or more features described herein.

## Claims

1. A fibre system for determining the shape of a flexible object, the system comprising having at least one coated optical fibre (21), the coated optical fibre having at least one optical fibre having at least one portion comprising a periodic distributed Bragg reflector (22) within the fibre, the at least one optical fibre being placed in and with a first flexible tube (32) positioned around, and wherein the first flexible tube further has a first coating (33) positioned around it.

2. The fibre system according to claim 1, wherein a second flexible tube (34) is positioned around the first coating and the second flexible tube has a second coating (35) positioned around it.

3. The fibre system according to claim 1 or claim 2, wherein there is more than one coated optical fibre within the fibre system.

4. The fibre system according to any preceding claim, wherein the first flexible tube is made from a shape memory alloy or a superelastic material.

5. The fibre system according to any preceding claim, wherein the first coating is made from at least one of: PTFE, graphene, thin carbon material, low-friction gel, low-friction fluid or a bearing system..

6. The fibre system according to any preceding claim, wherein the second or subsequent flexible tubes are made from a shape memory alloy or a superelastic material.

7. The fibre system according to any preceding claim, wherein the second coating is made from at least one of: PTFE, graphene, thin carbon material, low-friction gel, low-friction fluid or a bearing system.

8. The fibre system according to any preceding claim wherein the first and/or second coating is directly applied to the first and/or second flexible tube.

9. The fibre system according to any preceding claim, wherein the first and/or second coating is a thin film applied over the first and/or second flexible tube.

10. The fibre system according to any preceding claim, wherein surrounding the at least one coated optical fibre is a flexible support structure (36).

11. The fibre system according to any preceding claim, wherein the tolerance between the fibre Bragg optical fibre and the first flexible tube is between 0.005 and 0.5 mm.

12. A flexible arm system comprising an arm having at least a first (51) and second (52) section linked by a joint, the joint being coupled to actuators to adjust the angle of the first section relative to the second section, and wherein through the first section and second section a fibre system according to claims 1-12 is passed, and wherein the fibre system is coupled to an interrogator.

13. The flexible arm robot according to claim 12, wherein there are provided a plurality of sections (53, 54) of fibre system and between each section of fibre system is provided a bush (55).

14. The flexible arm robot according to claim 13, wherein the bush is made from at least one of Nitinol, copper or aluminium, plastic materials, 3D printable materials such as liquid resin materials or thermoplastics..

15. The flexible arm system according to any one of claims 12-14, wherein the fibre is integrated into the inner or outer structure of the flexible arm.
